# EUROPÄISCHE PATENTSCHRIFT

(11) **EP 0 453 728 B1**
(45) Veröffentlichungstag und Bekanntmachung des Hinweises auf die Patenterteilung: **06.11.1996**
(21) Anmeldenummer: 91102724.1
(22) Anmeldetag: 25.02.1991
(51) Int. Cl.: A61L 15/28, A61L 2/18

(54) **Verfahren zur Wiederaufbereitung von zellulosehaltigen Fasern und Saugkörper für medizinische oder hygienische Produkte**
Method for recycling cellulose fibres and medical or hygienic absorbent articles
Procédé de recyclage de fibres cellulosiques et des articles médicaux ou hygiéniques absorbants

(30) Priorität: 26.02.1990 DE 4005935
(43) Veröffentlichungstag der Anmeldung: 30.10.1991
(73) Patentinhaber: Paul Hartmann Aktiengesellschaft, D-89522 Heidenheim (DE)
(72) Erfinder: Zach, Siegfried, W-7920 Heidenheim (DE); Malowaniec, Krzystof D., Dipl.-Ing., W 7920 Heidenheim (DE)
(74) Vertreter: Becker, Maria, Dipl.-Phys.

(56) Entgegenhaltungen:
- DE-A- 2 710 806
- FR-A- 2 293 523

## Beschreibung

Die Erfindung betrifft ein Verfahren zur Wiederaufbereitung von zellulosehaltigen Fasern gemäss dem Oberbegriff des Patentanspruchs 1 und Saugkörper für medizinische oder hygienische Produkte, wie Windein, Saugeinlagen, Kompressen, Damenbinden, Tampons, Tamponaden usw., aus zumindest teilweise nach diesem Verfahren aufbereiteten, zellulosehaltigen Fasern.

Die für derartige hygienische und medizinische Saugkörper verwendeten Faserstoffe werden weitgehend aus Holz gewonnen. Das Holz besteht etwa zur Hälfte aus Zellulosefasern und zur Hälfte aus Lignin, einer amorphen Substanz, die als Bindesubstanz die Fasern zusammenhält. Bei der Herstellung von Zellstoff wird das Lignin in einem Kochprozess nach Durchführung eines Sulfat- oder Sulfit-Verfahrens verflüssigt und ausgespült.

Nach sorgfältigem mehrstufigem Waschen des so gewonnenen Faserstoffes erhält man eine "ungebleichte Zellulose". Die Fasern sind wie bei einfachem Packpapier oder Karton braun. Diese Färbung ist verursacht durch Reste von Lignin oder deren Abbauprodukten, die an der Faseroberfläche haften.

Für die Verwendung der Zellulosefasern für hygienische und medizinische Zwecke werden die Fasern gebleicht und die die reine Zellulosefaser noch ummantelnden, zum Teil farbigen Begleitsubstanzen beseitigt. Die Bleiche erfolgt in einem mehrstufigen Verfahren. In einer klassischen, fünfstufigen Bleichsequenz werden wechselweise Chlor- und Alkalisierungsbehandlungen durchgeführt. In moderneren Bleichverfahren wird eine gekürzte klassische Sequenz durch Chlordioxid- und/oder Sauerstoffstufen ergänzt.

Da die Chlorbleiche die Umwelt, insbesondere die Abwässer, stark belastet, ist es bekannt, die Bleiche der Zellulosefasern ohne den Einsatz von Chlor und bei vermindertem Einsatz von Chlordioxid durchzuführen. Man setzt aus diesem Grunde als Bleichmittel Sauerstoff ein, da hierdurch das Abwasser wesentlich weniger belastet wird.

Nach der Bleiche werden die Fasern in einem mehrstufigen Waschprozess gereinigt und anschliessend getrocknet.

Die gewonnene Fasermenge entspricht in etwa 40 bis 50 % der ursprünglich eingesetzten Holzmasse.

Eine weitere Faserart ist das sog. "CTMP" = "Chemo-Thermo-Mechanischer-Pulp". CTMP wird, wie Zellulose, aus Holz gewonnen. Dabei werden die Holzstücke mit Lauge und Dampf erweicht und anschliessend gemahlen. In einem solchen Prozess wird die Faser von dem Lignin-Mantel nicht befreit. Die Ausbeute dieser Faser aus der Holzmasse liegt damit wesentlich höher, nämlich bei ca. 95 %. Eine nachfolgende Bleiche ist bei CTMP nicht möglich, da sie das Lignin abbauen und damit die Wirtschaftlichkeit des Prozesses verringern würde. Aus diesem Grunde wird der CTMP-Stoff nur auf seiner Faseroberfläche mit Peroxid aufgebleicht. Allerdings ist diese Aufhellung reversibel, da das Produkt bei Einwirkung von Sonnenstrahlung sehr schnell vergilbt. Diese Eigenschaft ist jedoch keinesfalls störend bei Saugkörpern für Hygieneprodukte, die mit einer Vliesstoffabdeckung versehen werden, wie z.B. Windeln. Dank der hohen Ausbeute und der chlorfreien Bleiche wird CTMP oft als ein umweltfreundlicher Faserstoff angesehen, der bei Hygieneprodukten der vorgenannten Art mit Vorteil einsetzbar ist.

Der Erfindung liegt die Aufgabe zugrunde, ein Verfahren zu schaffen, mit welchem Sekundärfasern in Saugkörpern so aufbereitet werden, dass sie für Hygiene- und medizinische Produkte in Saugkörpern verwendbar sind. Als Sekundärfasern sollen solche verstanden werden, die bereits mindestens einmal verarbeitet bzw. verwendet worden sind und dann, z.B. aus Altpapier, wiedergewonnen werden.

Übliche Sekundärfasern eignen sich nicht für den Einsatz in Hygieneprodukten, da sie normalerweise kontaminiert vorliegen und eine "verhornte", d.h. teilweise flüssigkeitsabweisende Oberfläche haben. Die Verhornung der Faseroberfläche ist eine Folge des Trocknungsprozesses bei der Herstellung des Papiers.

Je öfter die Fasern einen Recycling-Prozess erfahren haben, umso hydrophober wird die Faseroberfläche.

Dieser Nachteil einer Verhornung der Faseroberfläche kann auch nicht in ausreichendem Maße durch das in der EP-PS 0 202 237 beschriebene Verfahren beseitigt werden. In diesem Verfahren wird lediglich durch Beigabe von Detergentien die Faser gewaschen und oberflächlich hydrophilisiert. Dadurch wird die hydrophobierende Wirkung der Verhornung nicht beseitigt, sondern nur teilweise abgeschwächt. Ein negativer Nebeneffekt dabei ist, dass in dem Faserstoff die Chemikalien (Detergentien) verbleiben, was insbesondere einen Einsatz in medizinischen Produkten nicht zulässt, jedoch auch in Hygieneprodukten nicht erwünscht ist.

Aus der DE-A 27 10 806 ist ein Verfahren zur Desinfektion von Altpapier bekannt, bei dem das Papier in einer wässrigen Lösung, der Wasserstoffperoxid zugegeben ist, zerkleinert und in einem mehrstufigen Prozess gereinigt und desinfiziert wird. In einem Ausführungsbeispiel aus der DE-OS 27 10 806, das nur zu Vergleichszwecken durchgeführt wurde, war die wässrige Lösung alkalisch.

Aus der FR-A 2,293,523 sind Verfahren zur Behandlung von Altpapier bekannt, bei denen einer wässrigen Lösung zum Auflösen des Altpapiers u.a. Chemikalien, wie Natriumhydroxid, Natriumperoxid, Kaliumsilikat, zugefügt werden.

Es ist nun Aufgabe der Erfindung, ein verbessertes Verfahren zur Wiederaufbreitung von zellulosehaltigen Fasern bereitzustellen, die dann als Sekundärfasern für den Einsatz in Hygiene- und medizinischen Produkten besonders geeignet sind, sowie Saugkörper bereitzustellen, die zumindest teilweise aus diesen Fasern bestehen.

Diese Aufgabe wird durch ein Verfahren mit den im Anspruchs 1 angegebenen Merkmalen, sowie durch einen Saugkörper gemäss den Ansprüchen 5 oder 6 gelöst.

Der Verfahrensablauf ist wie folgt:

Sekundäre Faserstoffe werden einer Aufbereitungsanlage in Ballen zugeführt. Vor der Bearbeitung werden die Ballen geöffnet und in einer Vorrichtung (Pulper mit Rotor) zu einer wässrigen Suspension aufgelöst. Die Konzentration Faser/Wasser beträgt vorzugsweise 1 bis 7 Vol%.

Die Suspension wird so lange gerührt, bis die Faser vereinzelt ist. Schwere Verunreinigungen werden dann als Sediment von der Suspension getrennt, andere, wie Draht, Schnüre usw., als sogenannter Schopf entfernt.

Die vorgereinigte Fasersuspension, die eine homogene Masse bildet, wird in gleicher oder nächster Stufe weiterverarbeitet. Die Konzentration kann geändert werden, indem über Siebe, Zentrifugalkräfte oder dergleichen eine Eindickung oder durch Zugabe von Wasser eine Verdünnung erfolgt.

Bei stärker verschmutzter Suspension kann mittels eines Sortierers oder durch Hydrozyklone (Cleaner) eine Reinigung erfolgen, ebenso in einem Sedimentationsbecken.

In gleicher oder in einer weiteren Reinigungsstufe werden Chemikalien zugegeben werden, welche die Oberfläche der Faser anlösen und dadurch erneuern. Besonders geeignet sind Chemikalien mit delignifizierender Wirkung.

Zur Desinfektion werden in einer der Reinigungsstufen Peroxide zusammen mit alkalisierenden Mitteln, wie z.B. NaOH, in die Suspension eingeleitet.

Der so behandelten Masse werden nach der Reinigung und Bleiche Papier-Bentonite zugegeben. Es handelt sich hierbei um chemisch modifizierte Aluminiumsilikate auf Montmorilloritbasis, die alkalisch oder sauer aktiviert sein können. Der Anteil der Bentonite sollte 2 - 10 Gew.% betragen. Es hat sich gezeigt, dass die Faserstrukturen hierdurch eine bessere Wasseraufnahmefähigkeit durch Vergrösserung der Oberflächen erhalten und somit Wasser schneller aufsaugen. Gerade für Saugkörper für hygienische Zwecke ist diese Eigenschaft wünschenswert.

Nach dieser Behandlung wird die Faserpulpe gewaschen und anschliessend entweder ohne weitere Zusätze oder mit einer wässrigen Suspension der Primärfaser oder aber mit Saugleistung verbesserneden Hilfsmitteln vermischt der Entwässerungsmaschine oder einer Karton-Maschine zugeführt. Dort wird eine Faserbahn gebildet, die dann noch getrocknet und auf richtige Breite zugeschnitten werden muss. Die fertige Faserbahn kann dann z.B. einer Windelmaschine als Rohstoff für den Saugkörper zugeführt werden.

Das Verfahren nach der Erfindung wird nachfolgend an einem Ausführungsbeispiel erläutert:

Als Ausgangsstoff dient Ballenware, die je zur Hälfte gemischt ist aus Abfällen der Type T 14 und der Sorte O 14. Nach einer von einem einschlägigen Gremium (Verband Deutscher Papierfabriken; Bundesverband Papierrohstoffe e.V.; Bundesverband der deutschen Entsorgungswirtschaft e.V.) herausgegebenen Terminologie wird unter T 14 ein Chromoersatzkarton verstanden mit leichtem Andruck oder unbedruckt weiß und farbig, während man mit O 14 holzhaltige, weiße Späne mit leichtem Andruck bezeichnet. Chromoersatzkarton wird aus einer Mischung von Zellulose- und Holzschliffaser hergestellt und findet Einsatz bei der Herstellung von Kartonagen für die Verpackungsindustrie. Das Altpapier O 14 besteht ausschliesslich aus sogenannten Illustrierten-Spänen. Es sind dies die weitgehend unbedruckten Randstreifen, die beim Beschneiden der bedruckten Illustriertenseiten in der Druckindustrie entstehen und dann als Abfall zu Ballen zusammengepresst werden.

Die Ballenware wird vorbereitet, indem sie von den Befestigungsdrähten befreit wird und in eine Stoffauflösevorrichtung (Pulper) eingebracht. Der Masse im Pulper wird Wasser zugegeben bis dass die Konzentration der Faserstoffe des Altpapiers im Wasser bei etwa 12 Gew.% liegt. Mittels Rotors wird die Masse zur Rotation gebracht und infolge Faser- zu Faserreibung eine etwa gleichmässige Suspension erreicht.

Die grobe Verschmutzung wird in Form eines Schopfes aus dem Pulp herausgezogen. Bei Entleerung des Pulpers wird die Suspension durch Zugabe von Wasser auf eine Konzentration von ca. 3 - 4 Gew.% verdünnt und dann einer Sortierstufe zugeführt. Die Sortierung erfolgt dabei durch Zentrifugalkräfte. Als erste Sortierstufe werden Drucksortierer mit Lochperforation eingesetzt. Dann werden die schwereren und leichteren Verunreinigungen entfernt und der entstandene Gutstoffstrom einem Entstipper zugeführt. Bei diesem handelt es sich um eine wie eine Mühle arbeitende Vorrichtung, in der die Stippen (Faserbündel) nochmals aufgeschlossen werden.

Die so aufbereitete Suspension wird dann der nächsten Sortierstufe, die aus einem Drucksortierer mit Schlitzperforation besteht, zugeführt. Danach kann im Normalfall die Suspension in eine Papiermaschine oder Kartonmaschine weitergeführt werden.

Zur weiteren Reinigung kann der aufbereitete Stoff noch einer Flotationsstufe zugeführt werden, in der ein Deinkingprozess stattfindet. Hierbei werden Druckfarben und ähnliche leichte Verunreinigungen entfernt.

Für den nach der Erfindung vorgesehenen Verwendungszweck ist eine Eindickung der Masse auf eine Konzentration von ca. 30 % vorgesehen. Die Masse wird anschliessend einer Sauerstoffbehandlung zugeführt, die in einem Sauerstoffturm stattfinden kann. Hierbei werden im Gegenstrom zur Masse alkalisierende Mittel mit einem Peroxid eingeschleust. In dieser Stufe findet einerseits eine Erneuerung der Faseroberfläche durch leichte Delignifizierung statt und anderereseits aber auch eine Desinfektion der Masse mit leichter Bleichwirkung, so dass nach dieser Behandlung der Faserstoff heller erscheint. Anschliessend wird die Stoffsuspension vorzugsweise nochmals ausgewaschen und auf eine übliche Konzentration verdünnt. Danach werden 2 - 10 Gew.% Bentonite zugesetzt.

Die Suspension wird dann der Karton-Maschine zugeführt. In dieser wird die Suspension über ein Sieb entwässert. Auf dem Sieb verbleibt eine Faserbahn, die in eine Nasspartie einer Presse überführt wird. In dieser wird sie durch Auspressen zwischen Filzen und Walzen mechanisch entwässert und dann einer Trockenpartie zugeführt, die aus mehreren Trockenzylindern bestehen kann. Das Material verlässt die Karton-Maschine getrocknet und abgekühlt als aufgerollte Bahn. Diese Bahn wird auf die erforderliche Breite zugeschnitte und als weiterverarbeitender Rohstoff an die Hygienieindustrie geliefert.

Es hat sich gezeigt, dass Saugkörper, die Fasermaterialien nach dem vorstehend aufbereiteten Verfahren enthalten, besonders gute Saugeigenschaften aufweisen, wenn die Saugkörper dieser Produkte ca. 50 - 70 % Frischfasern und 50 - 30 % nach vorstehend beschriebenen Verfahren aufbereitete Sekundärfasern enthalten.

## Patentansprüche

1. Verfahren zur Wiederaufbereitung von zellulosehaltigen Fasern für die Verwendung in Saugkörpern für medizinische oder hygienische Produkte, wie Windeln, Saugeinlagen, Kompressen, Damenbinden, Tampons, Tamponaden, bei dem die Behandlung der Fasern in einer wässrigen Suspension erfolgt, der Suspension die Faseroberfläche aufschliessende, insbesondere desinfizierende Chemikalien beigegeben werden und eine Reinigung der zellulosehaltigen Fasern in der wässrigen Suspension in mehreren Stufen erfolgt, wobei in einer Reinigungsstufe Peroxide in einem alkalischen Medium eingesetzt werden, dadurch gekennzeichnet, dass nach der Reinigung und Bleiche (Desinfektion) 2 - 10% Bentonite zugesetzt werden.

2. Verfahren nach Anspruch 1, dadurch gekennzeichnet, dass die Reinigung der zellulosehaltigen Fasern in mindestens einer Reinigungsstufe nach einer Eindickung der Suspension durch Beigabe von Sauerstoff oder Sauerstoff freisetzenden chemischen Verbindungen erfolgt.

3. Verfahren nach einem der vorhergehenden Ansprüche, dadurch gekennzeichnet, dass die aufzubereitenden zellulosehaltigen Fasern im wesentlichen aus im wesentlichen unbedruckten Papierabfällen hälftig aus Abfällen bestehen.

4. Verfahren nach Anspruch 3, dadurch gekennzeichnet, dass es sich um Papierabfälle der Type O 14 (holzhaltige, weisse Späne mit leichtem Andruck) und der Type T 14 (Chromoersatzkarton) handelt.

5. Saugkörper für medizinische oder hygienische Produkte, wie Windeln, Kompressen, Damenbinden, Tampons, Tamponaden, dadurch gekennzeichnet, dass er mindestens teilweise sekundäre Recyclingfasern, die nach einem der Verfahrensansprüche 1 bis 4 aufbereitet sind, enthält.

6. Saugkörper nach Anspruch 5, dadurch gekennzeichnet, dass er 50 - 70 %, Frischfasern und 50 - 30 % Sekundärfasern enthält.

## Claims

1. Process for the reprocessing of fibres containing cellulose for use in absorbent bodies for medical or sanitary products, such as diapers, absorbent liners, compresses, sanitary napkins, tampons, tamponades, comprising the steps of treating the fibres in an aqueous suspension, adding to the suspension chemicals that disintegrate, specifically disinfect, the fibre surface and cleaning the fibres containing the cellulose in the aqueous suspension in several steps, one of them employing peroxides in an alkaline medium, characterized in that 2 to 10 % of betonites are added after the cleaning and bleaching (disinfecting) steps.

2. Process according to claim 1, characterized in that cleaning of the fibres containing cellulose is effected in at least one cleaning step after the suspension has been thickened by the addition of oxygen or oxygen-releasing chemical compounds.

3. Process according to any of the preceding claims, characterized in that substantially half of the fibres containing cellulose, that are to be reprocessed, consist of substantially unprinted paper waste.

4. Process according to claim 3, characterized in that the paper waste is of the type O 14 (white chips containing wood with slight impression) and type T 14 (imitation chromo board).

5. Absorbent body for medical or sanitary products, such as diapers, compresses, sanitary napkins, tampons, tamponades, characterized in that it contains, at least in part, secondary reclycled fibres that have been processed according to one of process claims 1 to 4.

6. Absorbent body according to claim 5, characterized in that it contains 50 to 70 % fresh fibres and 50 to 30 % secondary fibres.

## Revendications

1. Procédé pour le retraitement de fibres à base de cellulose pour l'utilisation dans des corps aspirants pour produits médicinaux ou hygiéniques, tels que couches, garnitures aspirantes, compresses, serviettes hygiéniques, tampons, tamponnades, pour lesquels les fibres sont traitées en suspension aqueuse à laquelle sont additionnés des substances chimiques, essentiellement désinfectantes, que désagrègent la surface des fibres et un nettoyage des fibres à base de solvant s'effectue dans la suspension aqueuse en plusieurs phases, des peroxydes dans un milieu alcalin étant utilisés dans une des phases de nettoyage, caractérisé en ce que 2 - 10% de bentonite sont additionnés après le nettoyage et le blanchiment (désinfection).

2. Procédé selon la revendication 1, caractérisé en ce que le nettoyage des fibres à base de cellulose dans au moins une des phases s'effectue, après un épaississement de la suspension, par addition d'oxygène ou de combinaisons chimique dégageant de l'oxygène.

3. Procédé selon l'une des revendications précédentes, caractérisé en ce que les fibres à base de cellulose à traiter consiste essentiellement à moitié en déchets de papier essentiellement non imprimé.

4. Procédé selon la revendication 3, caractérisé en ce qu'il s'agit de déchets de papier du type O 14 (copeaux blancs avec bois et légère impression) ou de type T 14 (carton blanchi pour boîtes plantes).

5. Corps aspirant pour produits médicinaux et hygiéniques, tels que couches, compresses, serviettes hygiéniques, tampons, tamponnades, caractérisé en ce qu'il contient au moins partiellement des fibres secondaires de recyclage traitées selon l'une des revendications de procédé 1 à 4.

6. Corps aspirant selon la revendication 6, caractérisé en ce qu'il contient 50 - 70 % de fibres fraîches et 50 - 30 % de fibres secondaires.
